# EUROPEAN PATENT SPECIFICATION

(11) **EP 1 982 736 B1**
(45) Date of publication and mention of the grant of the patent: **18.11.2015**
(21) Application number: 06766889.7
(22) Date of filing: 19.06.2006
(51) Int. Cl.: A61M 1/16, A61K 45/06, A61K 31/7004, A61K 33/00, B01D 61/02, C02F 1/00, C02F 1/28, C02F 1/44, C02F 9/00, C02F 1/461, C02F 103/02

(54) **WATER FOR PREPARING DIALYSATE AND DIALYSATE AND METHOD OF PRODUCING DIALYSATE USING THE SAME, AND DIALYZER**
WASSER ZUR HERSTELLUNG EINES DIALYSATS UND DIALYSAT UND VERFAHREN ZUR HERSTELLUNG DES DIALYSATS DAMIT UND DIALYSEGERÄT
EAU SERVANT À PRÉPARER UN DIALYSAT ET DIALYSAT ET PROCÉDÉ DE PRODUCTION D'UN DIALYSAT UTILISANT CELLE-CI ET DIALYSEUR

(30) Priority: 21.04.2006 JP 2006118165
(43) Date of publication of application: 22.10.2008
(73) Proprietor: Nihon Trim Co., Ltd., Osaka-shi, Osaka 5310076 (JP)
(72) Inventor: KABAYAMA, Shigeru, Osaka-shi, Osaka 531-0076 (JP); MORISAWA, Shinkatsu, Osaka-shi, Osaka 531-0076 (JP)
(74) Representative: Willett, Christopher David
(86) International application number: PCT/JP2006/312225
(87) International publication number: WO 2007/122740

(56) References cited:
- EP-A1- 0 826 636
- WO-A1-2004/014355
- WO-A1-2006/019855
- CA-A1- 2 406 476
- JP-A- 09 077 672
- JP-A- 2005 329 061
- JP-A- 2006 028 079
- US-A1- 2005 020 507
- SUHAIL AHMAD: "Essentials of water treatment in hemodialysis", HEMODIALYSIS INTERNATIONAL, vol. 9, 1 April 2005 (2005-04-01), pages 127-134, XP002717643,

## Description

### Technical Field

The present invention relates to a dialysis solution and a method of producing a dialysis solution.

### Background Art

Dialysis is known as one effective treatment for the renal insufficiency patient whose kidney functioning is so degraded that he/she cannot urinate to adjust the amount of moisture and to remove metabolic toxic substances including body waste such as urea. The dialysis treatment is mainly divided into hemodialysis (HD) and peritoneal dialysis. Hemodialysis is a treatment including the steps of drawing blood outside the body using a blood pump, bringing the blood in contact with a dialysis solution through a dialyzator (dialyzer) to remove metabolic toxic substances and moisture taking advantage of diffusion based on the concentration gradient, and returning the purified blood (blood) into the body continuously. Peritoneal dialysis is a treatment of introducing the dialysis solution into the peritoneal cavity to remove metabolic toxic substances in the body and moisture through the peritoneal membrane.

The dialysis solution includes various electrolytes having a concentration close to that of normal blood. For example, a bicarbonate type dialysis solution for hemodialysis corresponds to a composition basically including ions of sodium, potassium, calcium, magnesium, chloride, acetic acid, bicarbonic acid and glucose. Such dialysis solution is prepared by diluting a liquid concentrate of high concentration. Since the bicarbonate may react with calcium ions and magnesium ions present in the bicarbonate dialysis solution to cause precipitation of insoluble substances, the dialysis liquid concentrate of the bicarbonate dialysis solution is generally realized by the two-component type, i.e. liquid A including electrolytic salt (salt such as of sodium, potassium, calcium, magnesium, chloride, acetic acid, and the like) and liquid B including sodium bicarbonate. Liquid A and liquid B are placed separately within the dialysis equipment, prepared at the time of usage by mixing/diluting to be used. For a dialysis liquid concentrate of the one-component type, there is known the acetic acid dialysis solution that does not contain bicarbonate.

In recent years, there has been known a method of preparing a dialysis solution by dissolving dialysis powder concentrate that is salt in powder form or granule form instead of the liquid concentrate of high concentration. The dialysis powder concentrate is formed of two agents, i.e. powder A including sodium chloride, potassium chloride, calcium chloride, magnesium chloride, acetic anhydride sodium and glucose (arbitrarily containing glacial acetic acid as a pH regulator), and powder B that is sodium bicarbonate powder. At the time of usage, the two agents are mixed/diluted with water to be prepared as a dialysis solution. There is also known the type formed of three agents, i.e. powder A-1 including sodium chloride, potassium chloride, calcium chloride, magnesium chloride, and acetic anhydride sodium, powder A-2 that is glucose powder, and powder B that is sodium bicarbonate powder. Further, there is known the type including liquid as one agent and powder or granule as the other agent (for example, the combination of liquid A including sodium, potassium, calcium, magnesium, chloride, and acetic acid ions as well as glucose, and powder B that is sodium bicarbonate powder.

For the dilution of the dialysis liquid concentrate or dialysis powder concentrate, purified water having impurities and foreign objects removed from raw water such as tap water is generally employed. Purification for preparing the water to be used for dilution includes the steps of removing contaminants and particles included in the raw water by a prefilter, removing the hardening component by softening equipment (for example, softening of the raw water by ion exchange), removing residual chlorine using an activated carbon device, removing trace metals including various metal ions using a reverse osmosis membrane, and the like.

The dialysis liquid concentrate or powder concentrate is generally subjected to heat sterilization in the production process thereof. At this stage, the glucose included in the dialysis liquid concentrate or powder concentrate is decomposed to produce glucose degradation products (GDP) such as glyoxal and/or methylglyoxal. Further, glucose degradation products are also generated by autoxidation of the dialysis liquid concentration or powder concentrate. The glucose degradation products will still be contained in the dialysis solution obtained by diluting the dialysis liquid concentrate or powder concentrate. Such a dialysis solution, when used in dialysis treatment, will become the cause of oxidative stress on the biological body of the dialysis patient to induce peritoneum tissue degeneration in association with glycation reaction (Advanced Glycation Endproduct: AGE). (Refer to Kidney International 51:182-186 (1997) (Non-Patent Document 1), Nephrol Dial Transplant, 14:1541 - 1549 (1999) (Non-Patent Document 2)). Furthermore, cell damage by oxidative stress is induced (refer to Biochemical Pharmacology 68:1433-1442 (2004) (Non-Patent Document 3)).

In order to suppress such adverse effects by the glucose degradation products, various devices such as the development of neutralized dialysis solution (for example, refer to Clinical Dialysis Vol. 19, No. 5, pp. 51-56 (2003) (Non-Patent Document 4)) have been made. However, the conventional devices are still insufficient. At the present stage, complete suppression of glucose degradation products is technically impracticable.
Patent Document 1: Japanese Patent Laying-Open No. 09-077672
Patent Document 2: Japanese Patent Laying-Open No. 10-118653
Patent Document 3: Japanese Patent Laying-Open No. 2003-175390
Non-Patent Document 1: Kidney International 51:182 - 186 (1997)
Non-Patent Document 2: Nephrol Dial Transplant, 14:1541 - 1549 (1999)
Non-Patent Document 3: Biochemical Pharmacology 68:1433 - 1442 (2004)
Non-Patent Document 4: Clinical Dialysis Vol. 19, No. 5, pp. 51- 56 (2003)

EP 0826636 concerns treatment of tap water by electrolysis.

WO 2004/014355 concerns a peritoneal dialysis solution comprising a pyruvate.

The paper "Essentials of water treatment in hemodialysis" in Hemodialysis International 2005; 9: 127-134 discusses water purity for dialysis.

### Disclosure of the Invention

### Problems to be Solved by the Invention

The present invention is directed to solve the problems set forth above. The object of the present invention is to provide a dialysis solution that can prevent the adverse effect of glucose degradation products on the biological body and a method of producing the dialysis solution.

### Means for Solving the Problems

The dialysis solution preparation water used in the present invention corresponds to water having a dissolved hydrogen concentration of 50 to 600 ppb, a pH of 7 to 10, and satisfying the water quality criterion defined at ISO 13959, wherein the water is used to prepare a dialysis solution by diluting a dialysis base agent including at least 50 ng/mL of a glucose degradation product.

The present invention provides a dialysis solution prepared by diluting a dialysis base agent including at least 50 ng/mL of a glucose degradation product, using dialysis solution preparation water having a dissolved hydrogen concentration of 50 to 600 ppb, a pH of 7-10, and satisfying the water quality criterion defined at ISO 13959.

Also disclosed is dialysis equipment including means for supplying dialysis solution preparation water having a dissolved hydrogen concentration of 50 to 600 ppb, a pH of 7-10, and satisfying the water quality criterion defined at ISO 13959, means for storing a dialysis base agent including at least 50 ng/mL of a glucose degradation product, and means for preparing the dialysis solution by diluting the dialysis base agent with said dialysis solution preparation water.

The means for supplying dialysis solution preparation water in the dialysis equipment preferably includes means for supplying raw water, means for electrolyzing the raw water, and means for subjecting cathode water obtained by electrolysis to a reverse osmosis membrane treatment.

The present invention further provides a method of producing a dialysis solution, wherein a dialysis base agent including at least 50 ng/mL of a glucose degradation product is diluted using dialysis solution preparation water having a dissolved hydrogen concentration of 50 to 600 ppb, a pH of 7 to 10, and satisfying the water quality criterion defined at ISO 13959.

### Effects of the Invention

The present invention can provide a dialysis solution that can prevent the adverse effect of glucose degradation products on the biological body, even if the dialysis solution is prepared using a dialysis base agent including at least 50 ng/mL of a glucose degradation product, and a method of producing the dialysis solution. Furthermore, the present invention can provide dialysis equipment for dialysis treatment using the dialysis solution of the present invention that can prevent the adverse effect of glucose degradation products on the biological body.

### Brief Description of the Drawings

Fig. 1 is a flowchart representing a favorable example of a method of producing a dialysis solution of the present invention.
Fig. 2 is a graph representing the results of evaluation experiments, employing hydrogen peroxide-luminol chemiluminescence, of the oxidation reducing capability for each dialysis solution preparation water of Example 1 and Comparative Example 1, wherein the vertical axis corresponds to the number of photons and the horizontal axis corresponds to time (second).
Fig. 3 is a graph representing the results of evaluation experiments, employing hydrogen peroxide-luminol chemiluminescence, of the oxidation reducing capability when MilliQ water is used as the test liquid for Reference Example 1, wherein the vertical axis corresponds to the number of photons and the horizontal axis corresponds to time (second).
Fig. 4 is a graph representing the results of evaluation experiments, employing hydrogen peroxide-luminol chemiluminescence, of the oxidation reducing capability for each dialysis solution of Example 2 and Comparative Example 2 as well as glucose solution of Reference Example 2, wherein the vertical axis corresponds to the number of photons and the horizontal axis corresponds to time (second).
Fig. 5 is a graph representing the CL total counts (vertical axis) of evaluation experiments, employing hydrogen peroxide-luminol chemiluminescence, of the oxidation reducing capability for each dialysis solution of Example 2 and Comparative Example 2.
Fig. 6 is a graph representing measurements of the concentration of contained glyoxal for various dialysis base agents, wherein the vertical axis corresponds to the glyoxal concentration (ng/mL) and the horizontal axis corresponds to the sample number.
Fig. 7 is a graph representing the CL total counts when the dialysis solution preparation water of Example 1 and Comparative Example 1 as well as ascorbic acid are added with respect to glyoxal of each concentration.

### Best Modes for Carrying Out the Invention

The dialysis solution preparation water used in the present invention has a dissolved hydrogen concentration in the range of 50 to 600 ppb, preferably 100 to 400 ppb, and particularly preferably 100 to 150 ppb. If the dissolved hydrogen concentration is below 50 ppb, the dialysis solution prepared using this dialysis solution preparation water cannot sufficiently prevent the adverse effect of the glucose degradation products on the biological body. If the dialysis solution preparation water includes dissolved hydrogen exceeding 600 ppb, the effect of preventing the adverse effect of the glucose degradation product on the biological body will not be improved any further. As used herein, dissolved hydrogen refers to H+, H●, H₂. Said dissolved hydrogen concentration refers to values measured through a dissolved hydrogen meter DH-35A (product of DKK-TOA Corporation).

The dialysis solution preparation water used in the present invention has a pH within the range of 7 to 10, preferably 8.5 to 9.5. If the pH is below 7, the effect of preventing the adverse effect of glucose degradation products on the biological body will be degraded. If the pH exceeds 10, the effect of preventing the adverse effect of the glucose degradation product on the biological body will not be improved any further. The pH of the dialysis solution preparation water used in the present invention corresponds to measurement readings using a pH meter (φ 260, product of Beckman Coulter, Inc.), with the pH electrode immersed in the dialysis solution preparation water.

The dialysis solution preparation water used in the present invention satisfies the water quality criterion defined at ISO 13959. Here, " satisfies the water quality criterion defined at ISO 13959" implies that the concentration of calcium, magnesium, potassium, sodium, arsenic, barium, cadmium, chromium, lead, mercury, selenium, silver, aluminum, chloramine, residual chlorine, copper, fluorine, nitrite nitrogen, sulfuric acid, zinc and tin does not exceed the criterion reference concentration shown in Table 1 set forth below.

**Table 1**

| Test Item | Criterion Concentration (mg/l=ppm) |
|---|---|
| calcium | 2 |
| magnesium | 4 |
| potassium | 8 |
| sodium | 70 |
| arsenic | 0.005 |
| barium | 0.1 |
| cadmium | 0.001 |
| chromium | 0.014 |
| lead | 0.005 |
| mercury | 0.0002 |
| selenium | 0.09 |
| silver | 0.005 |
| aluminum | 0.01 |
| chloramines | 0.1 |
| residual chlorine | 0.5 |
| copper | 0.1 |
| fluorine | 0.2 |
| nitride nitrogen | 2 |
| sulfuric acid | 100 |
| zinc | 0.1 |
| tin | 0.1 |

Confirmation of the dialysis solution preparation water used in the present invention satisfying the water quality criterion defined at ISO 13959 can be made by measuring respective concentrations of calcium, magnesium, potassium, sodium, arsenic, barium, cadmium, chromium, lead, mercury, selenium, silver, aluminum, chloramine, residual chlorine, copper, fluorine, nitrate nitrogen, sulfuric acid, zinc, and tin by means of the atomic absorption spectrophotometry, ICP atomic emission spectrometry, ICP mass spectrometry, reduction vaporized atomic absorption spectrophotometry, ion chromatography, and the like.

The dialysis solution preparation water used in the present invention is characterized in that it has a dissolved hydrogen concentration and pH within the specified ranges set forth above, satisfying the water quality criterion defined at ISO 13959, and used to prepare a dialysis solution by diluting a dialysis base agent including at least 50 ng/mL of a glucose degradation product. "Dialysis base agent" encompasses various dialysis liquid concentrates and powder concentrates used to prepare a dialysis solution, including those types having one agent in liquid form and another agent in powder form. A glucose degradation product refers to a substance generated by the decomposition of glucose basically contained in the dialysis base agent, and includes, for example, glyoxal, methylglyoxal, and the like. Even if the concentration of the glucose degradation products in the dialysis base agent is equal to or more than 50 ng/mL, the dialysis solution preparation water of the present invention is effective to significantly reduce oxidative stress caused by oxidation of the glucose degradation product when dialysis treatment is conducted using a dialysis solution prepared by diluting the dialysis base agent therewith. Side reactions such as peritoneum tissue degradation and cell damage caused by oxidative stress occurring in the patient undergoing dialysis can be prevented. The content (concentration) of the glucose degradation product in the dialysis base agent can be quantified by GC/MS, after the steps of, for example, directly adding pentafluorobenzylhydroxylamine (PFBOA) hydrochloride into a water sample rendered acid to obtain a derivative, decomposing excessive PFBOA with sulfuric acid, extraction by hexane, and dehydrating the extracted solution.

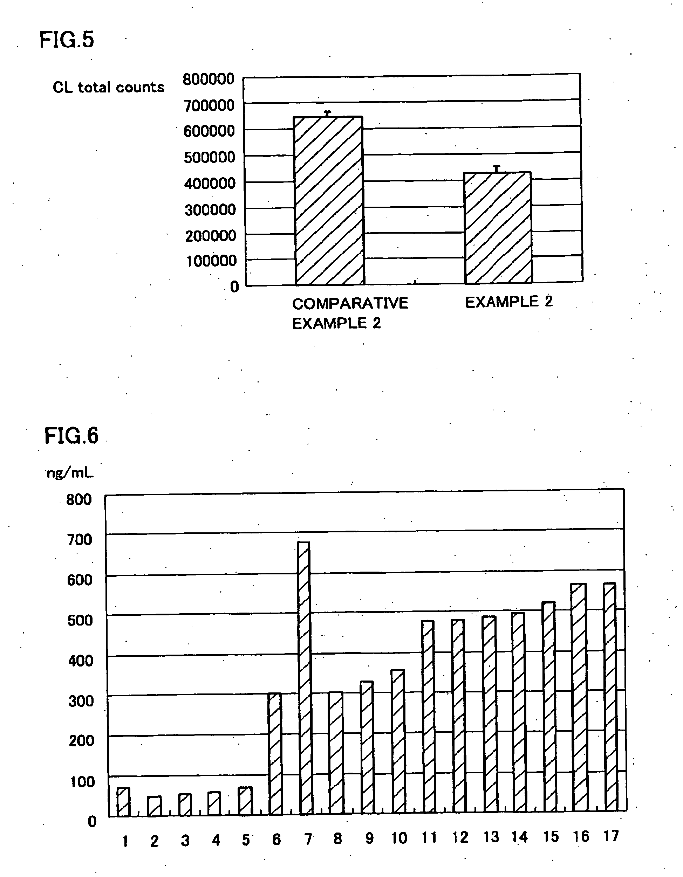

The dialysis base agent is generally subjected to heat sterilization in the production process thereof, during which glucose in the dialysis base agent is partially decomposed to generate glucose degradation products, as mentioned before. Further, glucose degradation products will be generated by autoxidation of the dialysis base agent, other than by heat sterilization. Thus, generation of glucose degradation products is inevitable in the dialysis base agent. Commercially-available dialysis base agents include a large amount of glucose degradation products, as compared to special grade glucose, as will be described afterwards with reference to Experiment 3. Comparing the powder-type dialysis base agent with the liquid-type dialysis base agent, it is identified that particularly many liquid-type dialysis base agents include glucose degradation products (refer to Experiment 3 and Fig. 6).

Thus, inclusion of glucose degradation products in the dialysis base agent is more or less inevitable. In the present invention, a dialysis solution is prepared by diluting the above-described dialysis base agent using dialysis solution preparation water having a dissolved hydrogen concentration and pH within the specified ranges set forth above, and satisfying the water quality criterion defined at ISO 13959. Therefore, oxidation of the glucose degradation products in the dialysis base agent can be reduced significantly to lower the oxidative stress on the biological body.

The ability of the dialysis solution preparation water used in the present invention set forth above to reduce the oxidation of the glucose degradation products in the dialysis base agent (oxidation reducing capability) can be evaluated by identifying the behavior of secondary fluorescence and the total chemiluminescence (CL) count employing, for example, hydrogen peroxide-luminol chemiluminescence (refer to the experiments set forth afterwards). Specifically, the method corresponds to the steps of exciting luminol that is a fluorescent reagent with hydrogen peroxide and evaluating the reduction in the oxidation of the substance having the oxidizing property taking advantage of emitted light at that time. When a substance having the oxidizing property and a substance having the oxidation reducing capability are present in a solution containing luminol and hydrogen peroxide, primary fluorescence of luminol caused by the reaction with hydrogen peroxide occurs, and then secondary fluorescence of luminol attributed to the substance having the oxidizing property is suppressed. As a result, the total CL count will become lower than the case where only a substance having the oxidizing property is present in a solution including luminol and hydrogen peroxide. Accordingly, the oxidation reducing capability of the substance having the oxidation reducing capability can be evaluated. The relevant evaluation test can be conducted conveniently using a CL analyzer (TOHOKU ELECTRONIC INDUSTRIAL CO., LTD).

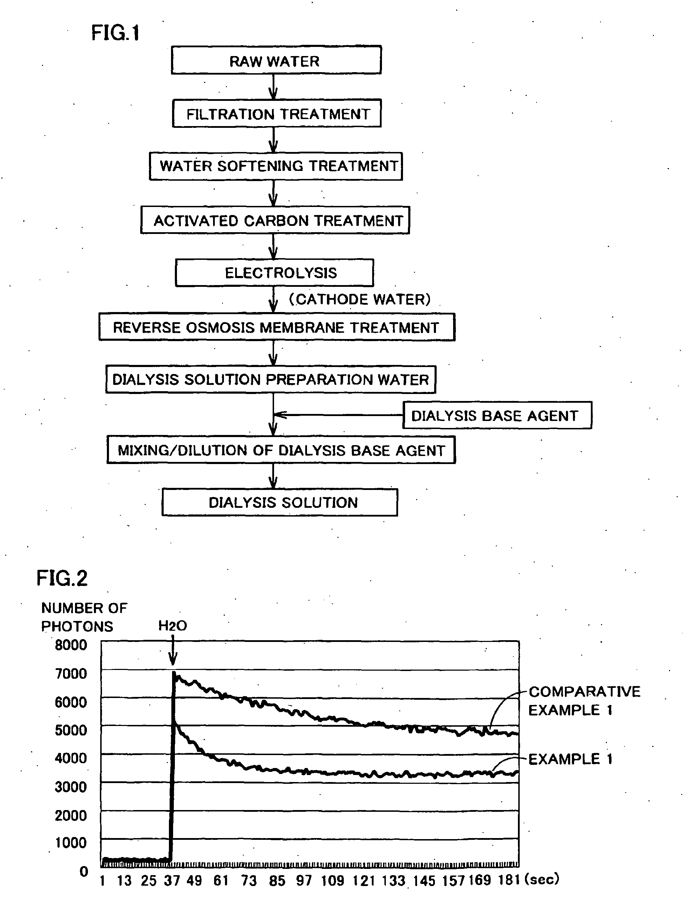

Fig. 1 is a flowchart representing a preferably example of a method of producing a dialysis solution of the present invention. The present invention provides a method of producing a dialysis solution characterized in that a dialysis base agent including at least 50 ng/mL of a glucose degradation product is diluted using dialysis solution preparation water having a dissolved hydrogen concentration of 50 to 600 ppb, a pH of 7 to 10, and satisfying the water quality criterion defined at ISO 13959. By virtue of the method of producing a dialysis solution of the present invention, a dialysis solution that can reduce oxidative stress on the biological body caused by glucose degradation products when applied to dialysis treatment can be produced conveniently, even in the case where a dialysis base agent including at least 50 ng/mL of a glucose degradation product is used.

Fig. 1 also represents respective steps in producing the dialysis solution preparation water used in the present invention used in the method of producing a dialysis solution of the present invention. The dialysis solution preparation water used in the present invention is not particularly limited as long as it has a dissolved hydrogen concentration and pH within the specified ranges set forth above, and satisfies the water quality criterion defined at ISO 13959. However, dialysis solution preparation water produced by electrolyzing daily life water such as tap water, well water or ground water as raw water, and subjecting the electrolytic reduced water (cathode water) obtained at the cathode side to a reverse osmosis membrane treatment can be used suitably. Similar to the conventional production of dialysis solution preparation water, preferably the water to be subjected to electrolysis is filtrated through a filter in advance, followed by water softening, and activated carbon treatment, as shown in Fig. 1. In this case, the order of carrying out the filtration treatment, water softening treatment, and activated carbon treatment is not particularly limited such as the order shown in Fig. 1.

In the example shown in Fig. 1, raw water such as tap water, well water, ground water, or the like is passed through a filter (prefilter) for filtration. The filter is not particularly limited, and an appropriate filter conventionally employed in producing dialysis solution preparation water (dilution water for dialysis) is suitable. Generally, a filter of 10 to 25 µm, for example, a 25-µm filter (product of Japan Water System), a 10-µm filter (product of Japan Water System), or the like is conveniently applicable. By the filtration treatment, coarse contaminants such as scale contained in the raw water (precipitation from the pipeline), and sand can be removed.

In the example of Fig. 1, the raw water is subjected to water softening after the filtration treatment. Water softening is the treatment of removing hardening components through substitution reaction caused by ion exchange from the raw water qualified as hard water including soluble solids (calcium ions, magnesium ions, and the like) identified as hardening components. An appropriate water softening device conventionally well known can be used without particular limitation for the water softening treatment. For example, MARK-915U (product of Japan Water System) is suitable.

In the example of Fig. 1, the raw water subjected to water-softening is next subjected to an activated carbon treatment. The activated carbon treatment removes residual chlorine, chloramine, organic substances, and the like included in the raw water through a physical adsorption by means of activated carbon which is a porous adsorbate. For the activated carbon treatment, an appropriate activated carbon processor conventionally well known can be used without particular limitation. For example, fibrous activated carbon MOF250C2 (product of Futamura Chemical Co., Ltd.) is suitable.

In the example shown in Fig. 1, the raw water subjected to activated carbon treatment is electrolyzed. Electrolysis can be conducted using an electrolytic water generator including a cathode chamber with a cathode and an anode chamber with an anode, separated from each other by a partition wall. In the electrolytic water generator, a cathode water outlet pipe from which cathode water (alkaline water) is drawn out is connected to the cathode chamber, and a drain pipe for discharging anode water (acidic water) outside is connected to the anode chamber. Each of the cathode chamber and anode chamber is connected with a supply pipe, configured to supply the raw water treated as set forth above. By the above-described electrolysis using the electrolytic water generator, electrolytic reduced water (cathode water) including dissolved hydrogen (H+, H●, H₂) can be obtained from the cathode. The cathode water obtained as set forth above has a dissolved hydrogen concentration and pH within the specified ranges set forth above.

The electrolytic water generator employed in the method of producing a dialysis solution of the present invention is not particularly limited, and an appropriate electrolytic water generator conventionally well known can be employed. For example, TRIMION HD-24k (product ofNihon Trim Co., Ltd.) is suitable. Although the conditions for electrolysis are not particularly limited, electrolysis is carried out conveniently under the conditions of 3 to 12A in current, 0.1 mV to 50 V in voltage, 4 to 35°C in temperature, and 1 to 24 L/min in flow rate from the standpoint of conveniently obtaining cathode water having the dissolved hydrogen concentration and pH within the specified ranges set forth above. It is to be noted that an appropriate electrolyte (sodium hydroxide, potassium hydroxide, sodium chloride, potassium chloride, hexachloroplatinic acid, or the like) can be added into the raw water in the electrolysis such that the raw water has an electric conductivity suitable for electrolysis (at least 100 µS/cm, more preferably 100 to 1000 µS/cm).

Following electrolysis, the cathode water obtained at the cathode side is subjected to a reverse osmosis membrane treatment. As used herein, a reverse osmosis membrane treatment refers to obtaining, when solutions of different concentration are present with a semi-permeable membrane therebetween, water permeating to the lower concentration side by applying pressure to the solution at the higher concentration side with respect to osmosis that is a phenomenon in which water moves from the solution of low concentration towards the solution of high concentration. By the reverse osmosis membrane treatment, impurities such as trace metals can be removed from the cathode water obtained by the series of treatments set forth above. Accordingly, water satisfying the water quality criterion defined at ISO 13959, in addition to the dissolved hydrogen concentration and pH of the specified ranges set forth above, can be obtained for the dialysis solution preparation water of the present invention. In the relevant reverse osmosis membrane treatment, an appropriate reverse osmosis (RO) device conventionally well known can be used without particular limitation. For example, HM500CX (product of Japan Water System) is suitable.

The procedures to produce the dialysis solution preparation water used in the present invention set forth above with reference to Fig. 1 is only a way of example. As long as the procedures of electrolyzing the raw water and applying a reverse osmosis membrane treatment are included, the remaining sequences may be replaced in order appropriately, or omitted. Further, an appropriate treatment conventionally employed to produce dialysis solution preparation water (dilution water for dialysis) may be combined (for example, filtration using a secondary filter prior to the reverse osmosis membrane treatment, sterilization through ultraviolet ray, or the like) to be replaced with or added to some of the procedures.

In the method of producing a dialysis solution used in the present invention, a dialysis solution is produced by mixing the dialysis solution preparation water produced as set forth above with a dialysis base agent for dilution. As mentioned before, the dialysis base agent encompasses the type completely in liquid form (dialysis liquid concentrate), and the type completely in powder or granule form (dialysis powder concentrate), as well as the type with one agent in liquid form and the other agent in powder or granule form.

The formula for mixing/diluting the dialysis base agent with the dialysis solution preparation water in the present invention is not particularly limited, and a preferable formula can be employed depending upon the dialysis base agent to be used. For example, based on an example of a dialysis liquid concentrate of the bicarbonate dialysis solution realized by the two-component type, i.e. liquid A including an electrolytic salt (including salt such as sodium, potassium, calcium, magnesium, chloride, acetic acid, or the like) and liquid B including sodium bicarbonate, the mixing formula of liquid A, liquid B, and the dialysis solution preparation water of the present invention (three-component mixture formula) includes: (1) first mixing liquid A into the dialysis solution preparation water, and then mixing liquid B; (2) first mixing liquid B into the dialysis solution preparation water, and then mixing liquid A; and (3) mixing liquid A, liquid B, and dialysis solution preparation water at the same time. In connection with the dialysis liquid concentrate of a bicarbonate type dialysis solution mentioned above, any of the mixture formulas of (1) to (3) set forth above will be generally adopted since direct mixture of liquid A and liquid B will cause reaction between the calcium chloride and magnesium chloride in liquid A with the sodium hydrogen carbonate in liquid B to cause precipitation. Although the mixing/dilution of the dialysis base agent may be carried out by any of these formulas in the method of producing a dialysis solution of the present invention, the formula of first mixing liquid B into the dialysis solution preparation water, and then mixing liquid A (formula (2)) is often used since concentration control is most feasible.

The ratio of diluting the dialysis base agent with the dialysis solution preparation water used in the present invention (dilution concentration) in the method of producing a dialysis solution of the present invention is adjusted to attain the dilution concentration set according to the dialysis base agent to be used. If the dilution concentration is too high in the obtained dialysis solution, there is a possibility of side effects such as a headache, cardiopalmus, elevation of blood pressure, disturbance in consciousness, or the like. If the dilution concentration is too low, there is a possibility of side effects such as numbness of the limbs, general malaise, precordial anxiety, rapid drop of blood pressure, disturbance in consciousness, or the like.

In the production method of the present invention for mixing/dilution of the dialysis base agent, adjustment is effected such that the obtained dialysis solution has an osmotic pressure ratio (namely, 0.95 to 1.00) effective to function as a dialysis solution. The osmotic pressure ratio of a dialysis solution refers to the ratio of the osmotic pressure measurement of the dialysis solution to the osmotic pressure of the physiological saline (theoretical value: 308 mOSm). The mixing/dilution of the dialysis base agent using the dialysis solution preparation water of the present invention in the method of producing a dialysis solution of the present invention should be carried out while adjustment is made such that the obtained dialysis solution has a pH and electrolytic concentration suitable as a dialysis solution.

The present invention provides a dialysis solution prepared by diluting a dialysis base agent including at least 50 ng/mL of a glucose degradation product using the dialysis solution preparation water set forth above, having a dissolved hydrogen concentration of 50 to 600 ppb, a pH of 7 to 10, and satisfying the water quality criterion defined at ISO 13959. Although the dialysis solution of the present invention includes at least 50 ng/mL of the glucose degradation product as described above, the oxidation of the glucose degradation products is reduced, so that the oxidative stress on the biological body during dialysis is reduced. The dialysis solution of the present invention is conveniently applicable to both hemodialysis and peritoneal dialysis.

The dialysis solution preparation water used in the present invention is not limited to water obtained by subjecting the cathode water obtained by electrolyzing raw water (preferably, raw water subjected to filtration, water softening, and activated carbon processing) to a reverse osmosis membrane treatment as described above with reference to Fig. 1, provided that the dialysis solution preparation water has a dissolved hydrogen concentration and pH within the specified ranges set forth above, and satisfies the water quality criterion defined at ISO 13959. For example, the dialysis solution preparation water of the present invention can be produced by a method including the steps of adding a hydrogen adsorbed metal colloid selected from the group consisting of platinum colloid, palladium colloid, vanadium colloid, iron colloid, and salicylic acid colloid into water, then generating dissolved hydrogen by hydrogen gas bubbling, mineral dissolution, ultrasonication, magnetization, physical innateness, microwave atomic vibration, photo irradiation, or the like, and then adjusting the dissolved hydrogen concentration, pH, and the impurity concentration such as of trace metals, or by a method including the steps of dissolving lithium and/or sodium, magnesium in acidic water, and then appropriately adjusting the dissolved hydrogen concentration, pH, and the impurity concentration such as of trace metals. In the aforementioned methods, the dissolved hydrogen concentration can be adjusted based on the intensity and time of, for example, hydrogen gas bubbling. The pH can be adjusted by controlling the added amount of, for example, sodium bicarbonate. The concentration of impurities such as trace metals can be adjusted by, for example, a reverse osmotic membrane treatment. Preferably, the dialysis solution preparation water of the present invention is produced through the series of procedures in the method of producing a dialysis solution of the present invention described with reference to Fig. 1.

The present invention may use dialysis equipment including means for supplying dialysis solution preparation water having a dissolved hydrogen concentration of 50 to 600 ppb, a pH of 7 to 10, and satisfying the water quality criterion defined at ISO 13959, means for storing a dialysis base agent including at least 50 ng/mL of a glucose degradation product, and means for diluting the dialysis base agent with said dialysis solution preparation water to prepare a dialysis solution. According to the dialysis equipment used in the present invention set forth above, a dialysis solution having the oxidation of the glucose degradation products suppressed can be produced, even if a dialysis base agent including at least 50 ng/mL of a glucose degradation product is used. Dialysis treatment (including both hemodialysis and peritoneal dialysis) can be carried out using such a dialysis solution without inducing side effects caused by oxidative stress on the patient.

The means for supplying dialysis solution preparation water in the dialysis equipment used in the present invention preferably includes means for supplying raw water, means for electrolyzing the raw water, and means for subjecting cathode water obtained by electrolysis to a reverse osmosis membrane treatment. Thus, dialysis equipment that can suitably carry out the method of producing a dialysis solution of the present invention according to the preferable embodiment set forth above can be realized. Further preferably, means for carrying out a filtration treatment, water softening treatment, and activated carbon treatment on the raw water are provided in the channel of the raw water between the means for supplying raw water and the means for electrolyzing the raw water.

Respective means set forth above in the dialysis equipment used in the present invention are not particularly limited, and may be realized by appropriately combining each means employed in an appropriate dialysis equipment conventionally well known and each device set forth in the method of producing a dialysis solution of the present invention (for example, a filter, water softening device, activated carbon processor, electrolytic water generator, reverse osmosis membrane device, and the like). The dialysis equipment of the present invention can be realized suitably based on a configuration similar to that of the dialysis equipment disclosed in Japanese Patent Laying-Open No. 09-77672 (Patent Document 1), for example, provided that the conditions for producing the dialysis solution preparation water are set at the above-described favorable conditions, and that a dialysis base agent including at least 50 ng/mL of a glucose degradation product is employed.

Although the present invention will be described in further detail based on experimental examples, it is to be understood that the present invention is not limited thereto.

### <Experiment 1>

Using tap water qualified as the raw water, a filtration treatment through a filter (25 µm-filter (product of Japan Water System) and 10-µm filter (product of Japan Water System)) was carried out, followed by a water softening treatment through a water softening processor (MARK-915U, product of Japan Water System), and an activated carbon treatment using fibrous activated carbon MOF250C2 (product of Futamura Chemical Co., Ltd).

The raw water subjected to the series of treatments set forth above was electrolyzed at the constant current of 6A under the conditions of 17°C in temperature and 7 L/min in flow rate using an electrolytic water generator (TRIMION HD-24k (Nihon Trim Co., Ltd.)). The cathode water obtained at the cathode side through electrolysis was subjected to the reverse osmosis membrane treatment through a reverse osmosis membrane device (MH500CX (product of Japan Water System)) to produce the dialysis solution preparation water of the present invention (Example 1). In a similar manner with the exception that electrolysis was not carried out, conventional dialysis solution preparation water (Comparative Example 1) was prepared.

Measurements on the dissolved hydrogen concentration and pH were obtained for the dialysis solution preparation water of Example 1 and Comparative Example 1. The dissolved hydrogen concentration was measured using a dissolved hydrogen meter DH-35A (product ofDKK-TOA Corp.). The pH was measured using a pH meter (φ 260, Beckman Coulter Inc.). The dialysis solution preparation water of Example 1 exhibited a dissolved hydrogen concentration of 170 ppb and a pH of 8.9, whereas the dialysis solution preparation water of Comparative Example 1 exhibited a dissolved nitrogen concentration of 0.1 ppb and a pH of 6.3. The concentration of the various components defined at ISO 13959 was measured through atomic absorption spectrophotometry, ICP atomic emission spectrometry, ICP mass spectrometry, reduction vaporized atomic absorption spectrophotometry, and ion chromatography on the dialysis solution preparation water of Example 1 and Comparative Example 1. All the results except for sodium, which was 2.0 mg/L, were below detection limit. None of the components exceeded the criterion level of the water quality criterion defined at ISO 13959.

The oxidation reducing capability was evaluated employing the hydrogen peroxide-luminol chemiluminescence on the dialysis solution preparation water of Example 1 and Comparative Example 1 (n = 3, respectively). First, 10 µl of 10 × PBS (Phosphate Buffered Saline), 90 µl of the test liquid (each dialysis solution preparation water of Example 1 and Comparative Example 1), and 1000 µl of lumino were mixed. At the elapse of 30 seconds, 1000 µl of hydrogen peroxide was further mixed therein. Then, the chemiluminescence (CL) count (number of photons) was identified continuously for 150 seconds. Measurement was initiated when the CL counts came to 200s per second (total time: 180 seconds). For the confirmation and measurement of the CL counts, a CL analyzer (product of TOHOKU ELECTRONIC INDUSTRIAL CO., LTD.) was employed.

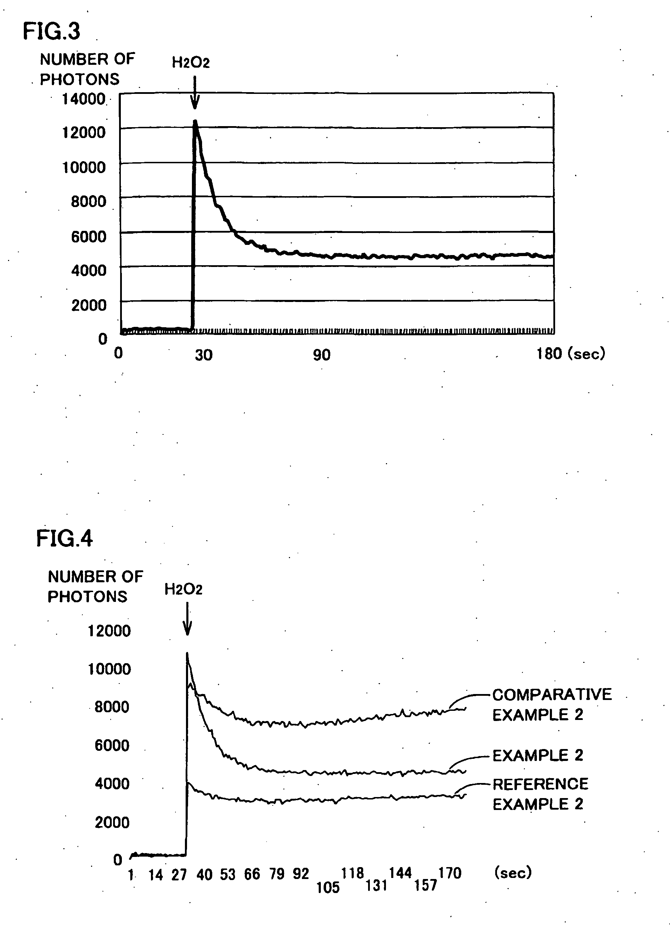

Fig. 2 is a graph representing the measured results for the dialysis solution preparation water of Example 1 and Comparative Example 1. The vertical axis represents the number of photons, and the horizontal axis represents time (second). Further, for Reference Example 1, the results of an experiment carried out in a manner similar to that set forth above employing MilliQ water as the test liquid is shown in Fig. 3 (n = 3). It is appreciated from Figs. 2 and 3 that the dialysis solution preparation water of Example 1 and Comparative Example 1 exhibited a low initial rise (primary fluorescence) of the CL count at the point of 30 seconds from the start of adding hydrogen peroxide, as compared to Reference Example 1. However, the subsequent CL count (secondary fluorescence) indicated a serial reduction equal between the dialysis solution preparation water of Comparative Example 1 and Reference Example 1. In contrast, secondary fluorescence could not be observed for the dialysis solution preparation water of Example 1, as compared to Comparative Example 1 and Reference Example 1. It was identified that the CL total count was also reduced significantly for the dialysis solution preparation water of Example 1. It was therefore appreciated that the dialysis solution preparation water of the present invention has superior oxidation reducing capability.

### <Experiment 2>

A dialysis base agent was diluted using the dialysis solution preparation water of Example 1 obtained in Experiment 1 to prepare a dialysis solution (Example 2). Similarly, a dialysis base agent was diluted using the dialysis solution preparation water of Comparative Example 1 to prepare a dialysis solution (Comparative Example 2). For the dialysis base agent, Kindaly solution AF- 3 (product of Fuso Pharmaceutical Industries, Ltd.) was used. Dilution was effected such that the ratio of liquid A: liquid B: dialysis solution preparation water was 1:1.26:32.74 to prepare respective dialysis solutions.

For the dialysis solution of Example 2 and Comparative Example 2, an evaluation experiment of the oxidation reducing capability was carried out in a manner similar to that of Experiment I(n = 3, respectively). Further, for Reference Example 2, a similar experiment was carried out (n = 3) for a special grade glucose solution (special grade glucose, product of Wako Pure Chemical Industries, Ltd.).

Fig. 4 is a graph representing the results of the evaluation experiments of the oxidation reducing capability employing the hydrogen peroxide-luminol chemiluminescence for the dialysis solution of Example 2 and Comparative Example 2 as well as for the glucose solution of Reference Example 2. The vertical axis represents the number of photons, and the horizontal axis represents time (second). Further, Fig. 5 is a graph representing the CL total counts (vertical axis) of evaluation experiments, employing hydrogen peroxide-luminol chemiluminescence, of the oxidation reducing capability for each dialysis solution of Example 2 and Comparative Example 2. It is appreciated from Figs. 4 and 5 that no secondary fluorescence is recognized for the dialysis solution of the present invention of Example 2, as compared to the dialysis solution of Comparative Example 2. The CL total count was also significantly reduced. It is appreciated that a behavior close to that of the glucose solution of Reference Example 2 was exhibited.

### <Experiment 3>

The concentration of glyoxal in the dialysis base agent was measured. For the dialysis base agent, six powder-type dialysis base agents (Samples 2-7) and ten liquid-type dialysis base agents (Samples 8-17) were used. For reference, the glyoxal concentration for the special grade glucose (Sample 1) was measured. The special grade glucose and dialysis base agents used are specifically set forth below.
Sample 1: Special Grade Glucose (product of Wako Pure Chemical Industries, Ltd.)
Sample 2: HYSORB-F (product of Ajinomoto Co., Inc.)
Sample 3: HYSORB-D (product of Ajinomoto Co., Inc.)
Sample 4: Kindaly 3E (product of Fuso Pharmaceutical Industries, Ltd.)
Sample 5: Kindaly 2E (product of Fuso Pharmaceutical Industries, Ltd.)
Sample 6: Kindaly 3D (product of Fuso Pharmaceutical Industries, Ltd.)
Sample 7: Kindaly 2D (product of Fuso Pharmaceutical Industries, Ltd.)
Sample 8: AK SOLITAFP (product of Ajinomoto Co., Inc.)
Sample 9: AK SOLITA DL (product of Ajinomoto Co., Inc.)
Sample 10: AK SOLITA FL (product of Ajinomoto Co., Inc.)
Sample 11: AK SOLITA DP (product of Ajinomoto Co., Inc.)
Sample 12: Kindaly Solution AF- 3P (product of Fuso Pharmaceutical Industries, Ltd.)
Sample 13 Kindaly Solution AF- 3 (product of Fuso Pharmaceutical Industries, Ltd.)
Sample 14: Kindaly Solution AF- 3S (product of Fuso Pharmaceutical Industries, Ltd.)
Sample 15: Kindaly Solution AF- 2P (product of Fuso Pharmaceutical Industries, Ltd.)
Sample 16: Kindaly Solution AF- 2S (product of Fuso Pharmaceutical Industries, Ltd.)
Sample 17: Kindaly Solution AF- 2 (product of Fuso Pharmaceutical Industries, Ltd.)

For the measurement of the glyoxal concentration of the special grade glucose and powder type dialysis base agents (Samples 1-7), dilution was effected so as to attain a concentration identical to that of the liquid type dialysis base agent, and measured by CG/MS. For the liquid-type dialysis base agents (Samples 8-17), the dialysis base agent was measured by GC/MS.

Fig. 6 is a graph representing results of Experiment 3, wherein the vertical axis corresponds to the glyoxal concentration (ng/mL) and the horizontal axis corresponds to the sample number. It is appreciated from Fig. 6 that, although there is no particular difference from the special grade glucose (Sample 1) for the powder-type dialysis base agents, some such as Samples 6 and 7 contained glyoxal of relatively high concentration. It was also identified that the liquid type dialysis base agents contained glyoxal of relatively high concentration as compared to that of special grade glucose. In view of the foregoing, it is expected that the glucose degradation products such as glyoxal included in the dialysis base agent still remain in the dialysis solution prepared by diluting the relevant dialysis base agent, which is the cause of oxidative stress on the patient undergoing dialysis treatment when such dialysis solution is used for dialysis treatment.

### <Experiment 4>

Using the dialysis solution preparation water of Example 1 and Comparative Example 1 obtained in Experiment 1, an evaluation experiment on the oxidation reducing capability for glyoxal was conducted. An evaluation experiment (n = 3) employing hydrogen peroxide-luminol chemiluminescence similar to that of Experiment 1 was carried out, provided that the test liquid used was prepared by adding 45 µl of each dialysis solution preparation water into 45 µl of glyoxal with the concentration of 1.0 mM, 10.0 mM and 100.0 mM such that the total amount of the test liquid was 90 µl. For a reference example, a similar experiment (n = 3) was carried out employing ascorbic acid (well known as a free radical scavenge substance). The test liquid was prepared such that the ascorbic acid was added to attain 1.0 µg/ml for 1.0M glyoxal, 2.5 µg/ml for 10.0M glyoxal, and 1.0 µg/ml for 100.0M glyoxal.

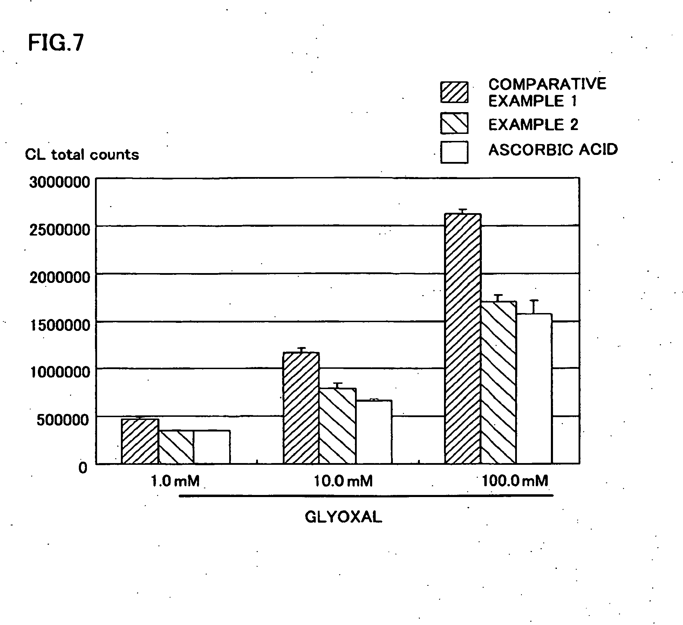

Fig. 7 is a graph representing the CL total counts when the dialysis solution preparation water of Example 1 and Comparative Example 1 as well as ascorbic acid are added with respect to the glyoxal of each concentration. It is appreciated from Fig. 7 that the CL total count was reduced significantly for the dialysis solution preparation water of the present invention as compared to the dialysis solution preparation water of Comparative Example 1. The CL total count could be reduced to a level similar to that of ascorbic acid. It is also appreciated from Fig. 7 that the dialysis solution preparation water of the present invention and ascorbic acid can have the CL total count reduced significantly as the concentration of glyoxal becomes higher.

It should be understood that the embodiments and examples disclosed herein are illustrative and non-restrictive in every respect. The scope of the present invention is defined by the terms of the claims, rather than the description above, and is intended to include any modification within the scope and meaning equivalent to the terms of the claims.

## Claims

1. A dialysis solution comprising water having a dissolved hydrogen concentration of 50 to 600 ppb and a pH of 7 to 10, **characterised in that** the water satisfies a water quality criterion defined at ISO 13959, and that the dialysis solution is prepared by diluting a dialysis base agent including at least 50 ng/mL of a glucose degradation product with the water.

2. A dialysis solution according to claim 1, wherein the water has a dissolved hydrogen concentration of 100 to 400 ppb.

3. A dialysis solution according to claim 2, wherein the water has a dissolved hydrogen concentration of 100 to 150 ppb.

4. A dialysis solution according to claim 1, 2 or 3, wherein the water has a pH of 8.5 to 9.5.

5. A method of producing a dialysis solution, wherein a dialysis base agent including at least 50 ng/mL of a glucose degradation product is diluted using dialysis solution preparation water having a dissolved hydrogen concentration of 50 to 600 ppb, a pH of 7 to 10, and satisfying a water quality criterion defined at ISO 13959.

6. A method according to claim 5, wherein the dialysis solution preparation water has a dissolved hydrogen concentration of 100 to 400 ppb.

7. A method according to claim 6, wherein the dialysis solution preparation water has a dissolved hydrogen concentration of 100 to 150 ppb.

8. A method according to claim 5, 6 or 7, wherein the dialysis solution preparation water has a pH of 8.5 to 9.5.

## Patentansprüche

1. Dialyselösung, umfassend Wasser mit einer Konzentration von gelöstem Wasserstoff von 50 bis 600 ppb und einem pH-Wert von 7 bis 10, **dadurch gekennzeichnet, dass** das Wasser ein Wasserqualitätskriterium gemäß ISO 13959 erfüllt und dass die Dialyselösung durch Verdünnen eines Dialysebasismittels, enthaltend mindestens 50 ng/ml eines Glucoseabbauproduktes, mit dem Wasser hergestellt wird.

2. Dialyselösung nach Anspruch 1, wobei das Wasser eine Konzentration von gelöstem Wasserstoff von 100 bis 400 ppb aufweist.

3. Dialyselösung nach Anspruch 2, wobei das Wasser eine Konzentration von gelöstem Wasserstoff von 100 bis 150 ppb aufweist.

4. Dialyselösung nach Anspruch 1, 2 oder 3, wobei das Wasser einen pH-Wert von 8,5 bis 9,5 aufweist.

5. Verfahren zur Herstellung einer Dialyselösung, wobei ein Dialysebasismittel, enthaltend mindestens 50 ng/ml eines Glucoseabbauproduktes, mit Wasser zur Herstellung einer Dialyselösung verdünnt wird, das eine Konzentration von gelöstem Wasserstoff von 50 bis 600 ppb und einen pH-Wert von 7 bis 10 aufweist und ein Wasserqualitätskriterium gemäß ISO 13959 erfüllt.

6. Verfahren nach Anspruch 5, wobei das Wasser zur Herstellung der Dialyselösung eine Konzentration von gelöstem Wasserstoff von 100 bis 400 ppb aufweist.

7. Verfahren nach Anspruch 6, wobei das Wasser zur Herstellung der Dialyselösung eine Konzentration von gelöstem Wasserstoff von 100 bis 150 ppb aufweist.

8. Verfahren nach Anspruch 5, 6 oder 7, wobei das Wasser zur Herstellung der Dialyselösung einen pH-Wert von 8,5 bis 9,5 aufweist.

## Revendications

1. Solution de dialyse comprenant de l'eau ayant une concentration d'hydrogène dissous de 50 à 600 ppb et un pH de 7 à 10, **caractérisée en ce que** l'eau satisfait un critère de qualité d'eau défini dans ISO 13959, et **en ce que** la solution de dialyse est préparée en diluant un agent de base de dialyse contenant au moins 50 ng/mL d'un produit de dégradation du glucose avec de l'eau.

2. Solution de dialyse selon la revendication 1, dans laquelle l'eau a une concentration d'hydrogène dissous de 100 à 400 ppb.

3. Solution de dialyse selon la revendication 2, dans laquelle l'eau a une concentration d'hydrogène dissous de 100 à 150 ppb.

4. Solution de dialyse selon la revendication 1, 2 ou 3, dans laquelle l'eau a un pH de 8,5 à 9,5.

5. Méthode de production d'une solution de dialyse, dans laquelle un agent de base de dialyse contenant au moins 50 ng/mL d'un produit de dégradation du glucose est dilué avec l'eau de préparation de la solution de dialyse ayant une concentration d'hydrogène dissous de 50 à 600 ppb, un pH de 7 à 10, et satisfaisant un critère de qualité d'eau défini dans ISO 13959.

6. Méthode selon la revendication 5, dans laquelle l'eau de préparation de la solution de dialyse a une concentration d'hydrogène dissous de 100 à 400 ppb.

7. Méthode selon la revendication 6, dans laquelle l'eau de préparation de la solution de dialyse a une concentration d'hydrogène dissous de 100 à 150 ppb.

8. Méthode selon la revendication 5, 6 ou 7, dans laquelle l'eau de préparation de la solution de dialyse a un pH de 8,5 à 9,5.
